# EUROPEAN PATENT SPECIFICATION

(11) **EP 3 229 669 B1**
(45) Date of publication and mention of the grant of the patent: **14.05.2025**
(21) Application number: 15867914.2
(22) Date of filing: 09.12.2015
(51) Int. Cl.: A61B 5/00, A61B 5/02, A61B 7/04, A61B 7/00, A61B 5/026, A61B 5/03

(54) **NONINVASIVE DETECTION OF HUMAN BRAIN CONDITIONS AND ANOMALIES**
NICHTINVASIVER NACHWEIS VON ERKRANKUNGEN UND ANOMALIEN DES MENSCHLICHEN GEHIRNS
DÉTECTION NON INVASIVE DE PATHOLOGIES ET D'ANOMALIES DU CERVEAU HUMAIN

(30) Priority: 09.12.2014 US 201414565337
(43) Date of publication of application: 18.10.2017
(73) Proprietor: Jan Medical, Inc., Mountain View, CA 94041 (US)
(72) Inventor: LOVOI, Paul A., Saratoga, CA 95070 (US); SCHUMACHER, Ray, Pine Valley, CA 91962 (US); MACVEAN, Charlie, San Diego, CA 92037 (US)
(74) Representative: SSM Sandmair
(86) International application number: PCT/US2015/064840
(87) International publication number: WO 2016/094586

(56) References cited:
- EP-B1- 0 541 671
- WO-A1-98/49934
- WO-A2-2014/167418
- US-A- 6 024 701
- US-A1- 2002 067 107
- US-A1- 2002 077 567
- US-A1- 2002 198 469
- US-A1- 2006 100 530
- US-A1- 2009 012 430
- US-A1- 2009 012 430
- US-A1- 2013 267 858
- YUKIO KOSUGI ET AL: "Detection and Analysis of Cranial Bruit", IEEE TRANSACTIONS ON BIOMEDICAL ENGINEERING, IEEE SERVICE CENTER, PISCATAWAY, NJ, USA, vol. BME-19, no. 3, 1 March 1987 (1987-03-01), pages 185 - 191, XP011174074, ISSN: 0018-9294

## Description

### Background of the Invention

This application is a continuation-in-part of application Serial No. 11/894,052, filed August 17, 2007, now U.S. Patent No. 8,905,932, which was a regular filing from provisional application No. 60/838,624, filed August 17, 2006.

The invention concerns detection of conditions of human vasculature or nonvascular brain tissue noninvasively, from outside the body, and in particular, locating aneurysms, vasospasm, partial or complete stenoses, ruptures, peripheral bleeding and other abnormal conditions pertaining to the cerebral vasculature and other cerebral tissue, profiling blood or other fluid flow through or around the cerebral vasculature and detecting and diagnosing abnormal cerebral conditions. The disclosure also encompasses mapping based on gathered data to provide a spatially resolved characterization of the major vasculature within the head. The system and method of the invention are also useful to detect vascular conditions in other parts of the body, especially the limbs and areas of other major vessels.

Stroke is a manifestation of injury to the brain which is commonly secondary to atherosclerosis or hypertension, and is the third leading cause of death in the United States. Stroke can be categorized into two types, ischemic stroke and hemorrhagic stroke. Additionally, a patient may experience transient ischemic attacks, which are in turn a high risk factor for the future development of a more severe episode.

Examples of ischemic stroke encompass thrombotic, embolic, lacunar and hypoperfusion types of strokes. Thrombi are occlusions of the arteries created *in situ* within the brain, while emboli are occlusions caused by material from a distant source, such as the heart and major vessels, often dislodged due to myocardial infarct or atrial fibrillation or carotid disease or surgical or percutaneous intervention. Thrombi or emboli can result from atherosclerosis or other disorders, for example, arteritis and lead to physical obstruction of arterial blood supply to the brain. Lacunar stroke refers to an infarct within non-cortical regions of the brain. Hypoperfusion embodies diffuse injury caused by non-localized cerebral ischemia secondary to low cerebral perfusion, typically caused by myocardial infarction, arrhythmia, blood loss or prolonged low blood pressure.

Hemorrhagic stroke is caused by intra cerebral or subarachnoid hemorrhage, i.e., bleeding in to brain tissue, following blood vessel rupture within the brain or venous thrombosis. Intra cerebral and subarachnoid hemorrhages are subsets of a broader category of hemorrhage referred to as intracranial hemorrhage.

Intra cerebral hemorrhage is typical due to chronic hypertension and a resulting rupture of an arteriosclerotic vessel. Stroke-associated symptom(s) of intra cerebral hemorrhage are abrupt, with the onset of headache and steadily increasing neurological deficits. Nausea, vomiting, delirium, paralysis, seizures and loss of consciousness are additional common stroke-associated symptoms.

In contrast, most subarachnoid hemorrhage is caused by head trauma or aneurysm rupture that is accompanied by highpressure blood release that also causes direct cellular trauma. Prior to rupture, aneurysms may be asymptomatic, or occasionally associated with headaches. However, headache typically becomes acute and severe upon rupture and may be accompanied by varying degrees of neurological deficit, vomiting, dizziness, and altered pulse and respiratory rates, phobophobia and severe headache and or neck stiffness.

Current diagnostic methods for stroke include costly and time-consuming procedures such as non-contrast computed tomography (CT) scans, electrocardiogram, magnetic resonance imaging (MRI) and angiography. Determining the immediate cause of stroke is difficult. CT scans can detect parenchymal bleeding greater than 5 mm and 95% of all subarachnoid hemorrhages. CT scans often cannot detect ischemic strokes until 6 hours from onset, depending on infarct size. CT only identifies 48% of acute strokes in the first 48 hours. MRI may be more effective than CT scan in early detection of ischemic from hemorrhagic stroke, but studies have shown that the superior data obtained from advanced imaging is more than offset by the degradation in patient condition due to the additional time necessary to complete the advanced imaging. Advanced imaging systems are not widely available at present and are limited to major hospital centers. Angiography is a definitive test to identify stenosis or occlusion of large and small cranial blood vessels, and can locate the cause of subarachnoid hemorrhages, define aneurysms, and detect cerebral vasospasm. It is, however, an invasive procedure and is also limited by cost and availability.

Immediate diagnosis and care of patients experiencing stroke can be critical. For example, tissue plasminogen activator (tPA) given within three hours of symptom onset in ischemic stroke is beneficial for selected acute stroke patients. In contrast, thrombolytics and anticoagulants are strongly contraindicated in hemorrhagic strokes. Thus early differentiation of ischemic events from hemorrhagic events is imperative. Moreover, delays in confirmation of stoke diagnosis and identification of stroke type limit the number of patients that may benefit from early intervention therapy. In addition, continuous monitoring of stroke patients is not possible with CT or MRI scanners, thus only one snapshot in time is available for diagnosis and treatment. Clinical observations are the basic tool that is used to monitor the progress of stroke patients.

Early detection of an aneurysm is beneficial as it can frequently be treated either by a surgical procedure of clip occlusion or by endovascular coil embolism. Presently, approximately three quarters of patients are treated with clip occlusion, the remainder with endovascular coil embolism. Either surgery, particularly the endovascular procedure, can by performed with low complication rate and high rate of success.

Once an aneurysm ruptures, however, the patient declines rapidly due to major brain injury, and over 50% of aneurysm rupture patients die acutely. Thus detection of at risk aneurysms is of great benefit. A physician faced with possible aneurysm warning signs must judge whether the symptoms warrant the trauma, expense and morbidity of contrast angiography.

Ferguson, in J. Neurosurg. 36:560-563 (1972), suggested detecting aneurismal signals by recording sounds from aneurysms exposed at operations using a cardiac phono-catheter.

Kosugi et al., in Stroke 14 (1) 37-42 (1983), disclosed the use of a "cement wall microphone", (contact accelerometer) in contact with the cranium and the teeth in an attempt to detect aneurysms. Charles P. Olinger et al., (U.S. 4,008,711) discloses a standard electronic low-noise microphone housed in an isolation chamber that detects sound waves from the patient's eyes. These sound waves are converted into an electrical signal that is passed though a final filter to a computer having a Fourier analysis capability.

Samir H. Manoli (U.S. 4,226,248) discloses a phonocephalographic device having a pair of ear insertable microphones that can detect sounds from the surface and cavities of the head. Data is output to a chart recorder or chart recorder. Sailor Mohler (U.S. 6,050,950) disclosed an acoustic means of recording heart sounds to determine blood pressure and diagnosing patients with vascular disease. Colin R. Taylor (U.S. 5,301,679) discloses using a microphone in contact with the body to record body sounds and using frequency domain and time domain sound amplitudes to provide diagnostic information on diseases of the body. Jorge M. Parra (U.S. 5,137,029) discloses acoustically coupling hydrophones to record acoustic signatures of the blood vessels of the eye to diagnose diseases of the eye. Laligam N. Sekhar et al. (U.S. 4,928,705) discloses a detector for brain aneurysm which comprises a multiple hydrophone sensor array for recording sounds emanating from the patient and a signal processing means that uses time domain and frequency domain analysis. Signal averaging over multiple heartbeats is disclosed to further improve signal processing.

David Lipson and Peter Forrest (U.S. 7,037,267) disclose a diagnostic method for diagnosis of the type of stroke that has occurred in a patient using ultrasonic signals directed into the brain. Norio Kawamura (U.S. 4,561,447) discloses an apparatus for detecting and displaying a pulse wave form from arterial vessels in the body irrespective of variation in pressing force of the sensing unit. Arminas Ragauskas et al. (U.S. 5,388,583) discloses an ultrasound noninvasive technique to derive time dependent characteristics of certain regions in the intracranium medium. Keith Bridger et al. (U.S. 6,6887,199) discloses a brain assessment monitor that uses a head-mounted passive acoustic sensor that generates a signal from pulsing blood flow through the patient's brain and an analysis means using time domain and frequency domain data to determine if the patient has had a brain injury or brain disease.

WO 98/49934 A1 discloses an intracranial pressure sensing system using accelerometers, failing to disclose using accelerometer signals to detect brain concussion.

Despite these known devices, there remains a need for an externally applied, noninvasive detector that is designed to more effectively record, analyze, localize and present, in a clinically useful manner, cerebral (including tissue and fluids between the vasculature and the skull) arterial and venous conditions. Several of the prior art methods have progressed to clinical trials and in some cases to FDA cleared devices. None of these devices provides reliable and clinically relevant information clinicians can rely upon to guide treatment, and none has made an impact on treatment of stroke. Devices and methods to date do not optimize the detection of useful signals from the brain or other parts of the body, have not localized the detected vascular conditions or tissue conditions and do not present the collected data in a clinically useful way. Seldom do patients present with a single simple condition but more typically they present with complex disease of the brain.

### Summary of the Invention

The current invention is a system and method (not part of the invention) for detecting a vascular condition or anomaly noninvasively in the human body, preferably the brain, by a collection of signal information received from the skull and originating from pulsing occurring at local, small regions of the vasculature. This is accomplished in a preferred embodiment by attaching, or contacting, one or an array of accelerometers or other sensors, to or against the head, and/or other points of interest, to a patient and recording signals from acceleration movements of the skull. The skull movements occur from heartbeat-induced repeated pulsing of blood flow into the vasculature of the brain and resultant expansion and subsequent contraction of the blood vessels which moves the brain tissue due to such pulsing, the brain tissue movement causing resultant displacement of the skull at measured positions on the skull. The accelerometer signals provide a signature unique to each patient and condition, and this can be called a "BrainPulse".

In one aspect of the disclosure the vibration signatures of blood vessel structures such as branches, aneurysms, stenoses, vasospasms or other structures using random, periodic, band limited or transient analysis provides a library for further processing. The library becomes part of a cerebral modeling arithmetic computer using basis functions, or other artificial neural network. The developed signature library is then used to facilitate localizing the origin of the recognized vascular feature; the localized feature is then presented to the physician in a clinically relevant manner.

The data to build the neural network (NN) and/or data store for correlation are obtained by acquiring data from patients with known pathologies as well as controls, and optionally with studies of constructed or cadaver vasculatures. The data are formatted according to the requirements of each processing method before being used for training or algorithm construction.

In one implementation, a system for noninvasively detecting conditions or anomalies in the vasculature or tissue of a patient's brain employs at least one and preferably a plurality of pressure pulse sensors adapted for positioning on the head of the patient at one or more selected locations so as to be directed toward the brain. The pressure pulse sensor or sensors are sufficiently engaged with the head of the patient to detect, to a high degree of accuracy, skull movements. A digitizer is connected to the pressure pulse sensors to convert the signals from multiple pressure pulse sensors essentially simultaneously. The digitized signals are sent to a comparitor capable of recognizing each of a plurality of particular data signatures as read by multiple pressure pulse sensors even at phase-shifted times among the sensors. This can involve various forms of analysis of the collected data, including filtering and analysis in the frequency domain.

It is the purpose of this disclosure to provide a continuous noninvasive method and equipment for detecting ischemic stroke, hemorrhagic stroke, aneurysms, stenosis, intracranial pressure and concussion (according to the invention) and identifying and localize multiple conditions within a single patient. It is also the purpose to monitor or screen high risk patients to detect conditions that may lead to a first or subsequent stroke, and further to detect other brain anomalies. These and other objects, advantages and features of the invention will be apparent from the following description of a preferred embodiment, considered along with the accompanying drawings.

### Description of the Drawings

Figure 1A is a plan and elevation view of sensors on a head.
Figure 1B is a block diagram of the steps of the invention.
Figure 2 is a block diagram of the sensor processing steps.
Figure 3 is a diagram of the correlation circuit.
Figure 4 is a diagram of a trigger pulse amplitude VS time.
Figure 5 is a diagram of sensors at various distances from a pulse source.
Figure 6 is a diagram of a trigger pulse and a received pulse at a distant sensor.
Figure 7 is the overlay of the trigger pulse and the received pulse shifted by time delta t₁.
Figure 8 is a diagram of a trigger pulse and a received pulse from a further sensor.
Figure 9 is a diagram of a trigger pulse and a received pulse from the furthest sensor.
Figure 10 is a table the time of first signal arrival from the trigger pulse for each sensor.
Figure 11 is a diagram of two intersecting spheres of different diameters.
Figure 12 is a diagram of three sensor signals shifted according to the table in Figure 10.
Figure 13 is a diagram of the sum of the three sensor signals in Figure 12.
Figure 14 is a received sensor signal as shown in Figure 6 and typical noise signal.
Figure 15 is a diagram of the sum of the noise signal and the signal from sensor 1.
Figure 16 is a diagram of the sum of noise signal and the signal from sensor 2.
Figure 17 is a diagram of the superposition of the noise signals from each of the three sensors.
Figure 18 is a diagram of the sum of the noise signals from each of the three sensors.
Figure 19A is a plan view in one plane of four sensors on a human head with the distances from a noise source to each sensor.
Figure 19B is a plan view in one plane of four sensors on a human head with several possible sources of sound indicated.
Figure 20 is a plan view of the head with sensors shown and the geometry of beam forming.
Figure 21 is a table giving the distances for four sensors and five beam forming position.

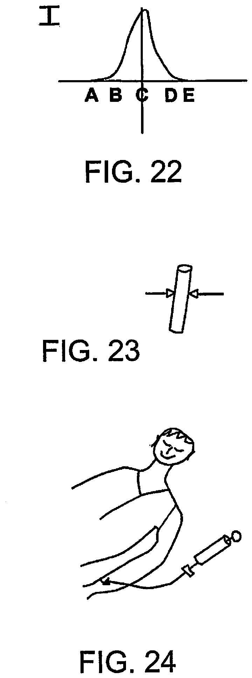

Figure 22 is a plot of the width of a blood vessel determined by beam forming.
Figure 23 is a diagram indicating the width of a blood vessel based on merging the NN output and the beam forming data.
Figure 24 is a figure of a patient being injected with ultrasound contrast agent.

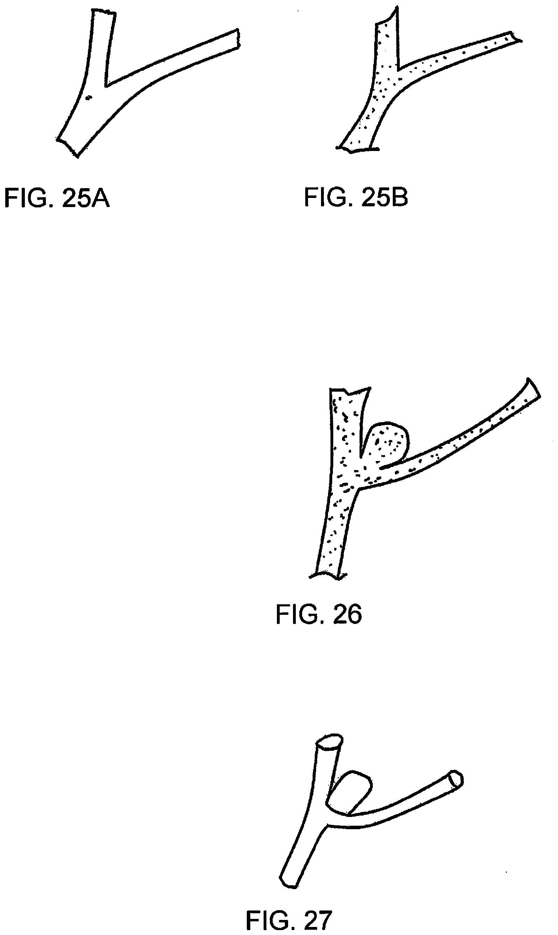

Figure 25A and 25B are figures of a branch in a blood vessel without and with ultrasound contrast.
Figure 26 is a figure of an aneurysm in the branch of a vessel as imaged with bursting bubbles.
Figure 27 is a figure of how an image of the vessel and aneurysm would be displayed after processing.

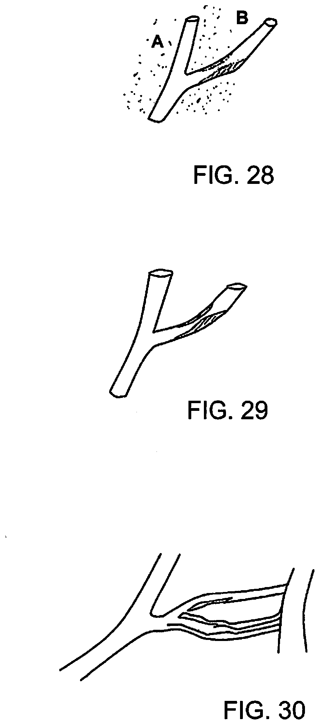

Figure 28 is a figure of a branching vessel and areas of perfusion and non-perfusion.
Figure 29 is a figure of a vessel blocked by a stenosis.
Figure 30 is a figure of a AVM (arterial venous malformation).

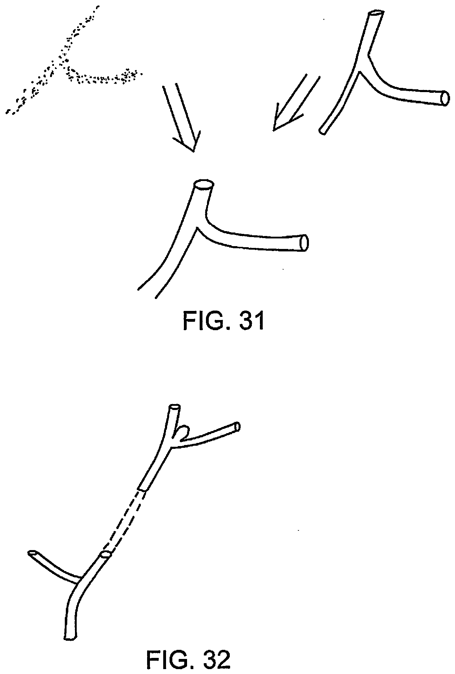

Figure 31 is a diagram of how bursting bubbles combine with a NN image to produce an improved image.
Figure 32 is a figure that depicts how two segments derived from imaging can be connected by knowledge of the anatomy.

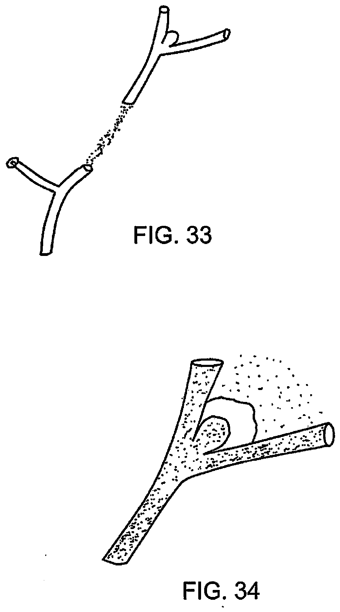

Figure 33 is a figure that depicts how two segments derived from imaging can be connected by bursting bubble data.
Figure 34 is a figure that depicts an area of pooled blood in proximity to an aneurysm.
Figure 35 is a flow diagram outlining the overall system.
Figures 36 and 36A are schematic views showing a human head with sensors engaged against the head at plural locations.
Figures 37 and 37A are schematic views indicating a photographic means of heating the sensors by reference to features of the patient's head.

### Description of Preferred Embodiments

### General Outline of Process

Periodic pulses from the heart produce waves of expanding blood vessels within the body. The tissue surrounding the blood vessels is displaced during the pulse and contracts again after the pulse. This displacement propagates outward from the blood vessel. In the case of the brain, this displacement reaches the skull and displaces the bone in response to the displacement of the blood vessel wall. Extremely sensitive accelerometers record this displacement. The character of the signal recorded by the accelerometers is dependent on the nature of the displacement caused by the blood pulse. At a restriction in the vessel the displacement before the restriction is larger than it would be without the restriction and the displacement beyond the restriction is less. The spatial distribution of displacement produces a different signature than that recorded by an unrestricted vessel. Likewise an aneurysm allows a circulation of blood within the bulb of the aneurysm during the pulse and produces a periodic signal that modifies the displacement signal. Other geometric arrangements of vessels likewise produce unique signatures in displacement.

The accelerometers are very sensitive, typically 500 mV/g or more. Such an accelerometer is a model from Dytran, of California. The invention detects and analyzes low-frequency signals from the accelerometers, below 500 Hz and for most analysis no higher than 100 Hz. Much analysis can be at 30 Hz or less.

### Limitations of Acoustic Signal Detectors

Accelerometers have a distinct advantage over microphones since microphones require acoustic signals to propagate through the soft tissue of the brain, conduct through the hard bone of the skull, again transfer to the soft tissue of the skin and finally be transferred to the microphone. Such devices have been most successful when attached to the teeth, positioned within the ear or focused on the eye sockets. Thus the use of microphones has been more successful when they can either have direct bone contact or look where there is no skull present. Accelerometers measure the displacement of the entire structure.

### Elimination of External Sources of Signal

The microphone is also very sensitive to sound signals produced from outside the body, such as sirens, talking, doors slamming and other sound signals. Accelerometers are much less sensitive to acoustic signals and generally are only sensitive to signals along the primary axis of the detector.

### Localization with Phase

The use of multiple accelerometers positioned around the head allows signals from different parts of the brain to be distinguished by location. Beyond just the relative magnitude of the signals from each of the sensors the phase relationship of the signals between sensors is used to determine the location of the displacement source. Since there are a multitude of signals produced during the pulse from different spatial positions and with the pulse traveling from the proximal to distal portions of the vasculature, a means to distinguish each of these signals is needed. This differentiation is accomplished by using *a priori* information about the head and the approximate positions of each of the accelerometers. For example signals cannot emanate from locations outside of the head nor at times that are not related to the blood pulse.

### Timing for localization

A gating sensor, an EKG electrode or photoplethysmogram (PPG) sensor, is used to provide timing information to the system. Since the heart rate varies in a given person both at rest and with exertion, the timing signal is used as an approximate starting point for timing the signal arrival at the sensors but cannot determine the pulse timing. Leeway is still needed as the intra pulse timing may be increasing or decreasing for each pulse.

### Localization Example

Signals data are captured from multiple sensors during a pulse. The signals from the multiple sensors are shifted in time by a guessed amount and then added together. The resulting signal is then analyzed either by direct amplitude, by Fourier transform or by another algorithms. This process is continued over the valid range of phases from each sensor. If a given signal is correlated with the signal from another sensor then the resulting combined signal will have an enhanced signature when the phases represent the location of the signal and not for surrounding phases within the allowable phase space. The quality of a signature can be quantified and that quantification can be established for each phase shift between sensors. As the phase is shifted over the possible range the figure of merit (quantified result of a single phase relationship) will increase and decrease. The peaks represent recognized signatures at localized positions. While this process is straightforward it is very processor intensive.

The allowable phase shifts for all the sensors can be calculated beforehand and this range used to limit the correlation space. The computational problem can be parsed such that parallel processors tackle the problem in parallel thus reducing the time from data collection to data presentation. It is important to note that once the initial data has been analyzed the relative phase between sensor data for the many signals within the brain is known, continued analysis of captured data is much less computational intensive. This *a priori* knowledge vastly reduces the computational requirements of the system and allows near real time presentation of the data. Current computers are certainly capable of completing the vast amount of computations in times suitable for clinical use and this will just improve as the speed and processing throughput of computers increases.

### Creation of Synthetic Image

The data needs to be presented in a recognized manner for the clinician to make use of it. By the nature of the data capture and processing, the most prominent structures are most readily identified and localized. This is not a complete map of the brain vasculature. Using *a priori* knowledge of the typical structure of the vasculature (and noting that there are significant deviations from the "typical" structure) the identified structures can be placed onto a cartoon of the entire vasculature, replacing the typical cartoon representations with improved representations based on the data analysis. This synthetic image is then presented to the clinician; as a 3D representation, a simulated CT or MRI scan or as a angiogram. As data continues to be acquired and processed the synthetic image can be updated.

### Ultrasound Bubbles

Ultrasound contrast agents are materials that can be injected as an IV to improve the imaging of blood vessels by ultrasound. The contrast consists of very fine particles and a method of attaching very small bubbles to the particle. To avoid any associated problems with embolisms the particle and bubbles together are smaller than a typical red blood cell thus allowing the bubbles to pass through from the arteries to the veins. These bubbles are also engineered to not join together as they are formed about a tiny particle rather than free standing bubbles that might have a tendency to enlarge by combining.

The purpose of injecting bubbles into the vasculature is to increase the ultrasonic contrast of the blood by adding well dispersed low density material. Contrast agent is rapidly eliminated in the blood by the breaking of the newly formed bubbles.

An unrecognized feature of the contrast agents is that as each bubble breaks it creates a disturbance within the vasculature which is highly localized. Ultrasound contrast media are already FDA cleared for use in stroke diagnostics and vessel imaging. To ensure the safety of injecting bubbles into the blood the bubble size is very well controlled. Bubbles of the same size have a well defined and distinctive signature when they break. This consistency allows for precise localization of the resulting signal. Since the bubbles are smaller than a red blood cell, the bubbles are able to perfuse from the arteries to the veins. The bubbles break randomly over a controlled period of time, typically five to 20 minutes after injection. If the amount of contrast media that is injected is reduced significantly from the amount typically given for ultrasound imaging the number of bubbles that break can be controlled to have, on average, a few milliseconds between bursts. Thus a system that is sensing the breaks from multiple sensors can localize the position of the bubble when it broke to a few mm or better resolution. Summing over a large number of events will produce a 3D map of where the bubbles have reached.

One of the major objectives of stroke treatment is determining whether a patient has a stroke at all and if they have had a stroke to differentiate an ischemic from a hemorrhagic. This differentiation is critical to caring for the patient. If clot dissolving drugs are used on a hemorrhagic patient the likely outcome is significantly worse, including death, over no treatment at all. Likewise not treating an ischemic stroke patient within the first four and one-half hours (for current drugs) of the stroke results in no treatment at all.

A 3D bubble map will show areas that are not perfused, an area with little or no bubbles showing up; the signature of a vessel blockage or ischemic stroke. Aneurysms, the typical cause of hemorrhagic strokes, will show up in both the direct sensor signals and in the 3D bubble map. A lack of perfusion in an area around the aneurysms would be a strong indicator of non flowing pooled blood. A perfused area distal to the aneurysm would be a further indication of no ischemic stroke. Thus the system would allow rapid, clinically relevant, differentiation of stroke type enabling treatment to be delivered in a timely manner.

### Merger of Data

The sensors deliver data on major unusual events in the vasculature. Merging this data into a "typical" 3D map of the complete vasculature ignores the real differences between individuals. It still provides clinically relevant data, such as lack of blood flow in an area of the brain and then a subsequently reperfusion of that area. This highlights the major advantage of having a continuous monitoring system over a snapshot monitoring such as CT, MRI or contrast angiography. Adding the bubble map would allow a complete map of the vasculature since the bubbles burst through out all the perfused vessels within the brain. Since the same sensors, and sensor locations, are used to produce both maps, the two data sets can be merged to form a single complete map of the vasculature.

In addition, this data set can be merged with the CT scan or other scans to combine the data from each to provide the clinician a better picture of the patients condition.

### Neural Network

The array of different accelerometer signatures produced by features within the vasculature is quite large due to the variable physiology of patients but there are generally only a few major underlying features. This type of data is ideal for using neural networks to identify the features causing the signature to be categorized. As the library of unique features and the associated signatures grows the neural network will improve in correctly identifying features in patients. Neural networks typically are trained by inputting a set of known inputs and known outputs and allowing the weights of the neural connections to change to optimize the matching of the inputs and outputs. When a new input is presented to the neural network the output closest to the input is given the highest output even if the input is not a perfect match to any of the training set. Another approach to determining the features within the accelerometer recordings that are associated with the brain abnormality is data mining. In this approach specific numerical features of the accelerometer recordings using the time domain, frequency domain or combinations of these, are used with data mining software such as CART (Salford Systems, San Diego, CA) to determine which variables best predict the abnormality from the accelerometer recordings.

### Beam Forming

In a process similar to localization previously discussed, beam forming varies the phase between data produced by different sensors. It is the purpose of beam forming to systematically vary the phase and retain the resulting signal. With small steps in the phase a very high spatial resolution map of the vasculature is produced. Beam forming is used when a feature of interest is localized and more detailed information is desired about that feature. Beam forming is computational intensive but lends itself to parallel processing and will be aided by improvements in processing power of both general purpose and special purpose processors.

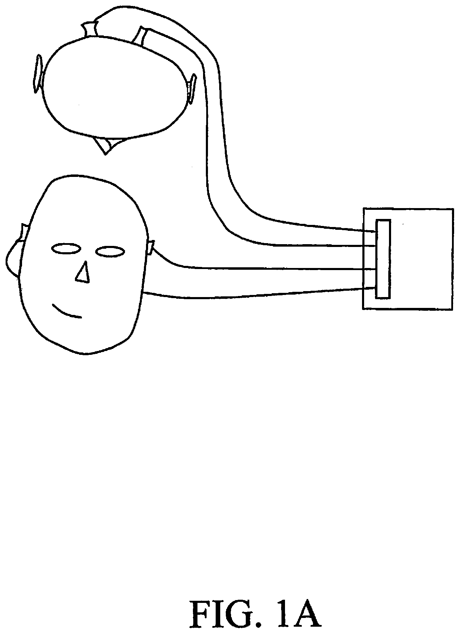

In the drawings, Figure 1A shows a patient with sensors attached to or engaged against various places on the head. The sensors are connected by cables, but could be connected wirelessly, to the analysis portion of the system. Note that useful data can be obtained using a single sensor, but multiple sensors at different locations are preferred. The sensors are engaged at bony cranium locations where the skull provides reliable detection of the motion of the brain due to the pulsatile flow of blood into the vasculature.

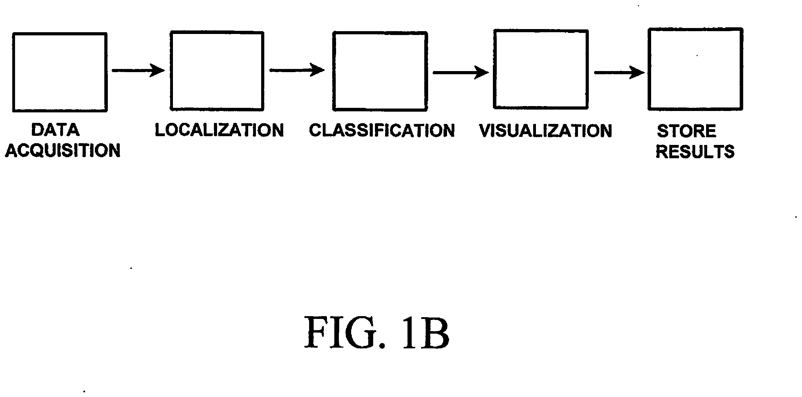

Figure 1B shows a block diagram of the analysis and visualization portion of the system. The signal arrives from each sensor and is conditioned and processed in the data processing block. The processed signal is digitized and processed in parallel through each of the algorithms that have been developed using either data mining software or neural networks. Each algorithm yields an output that relates the current subject's results to the results of many other subjects with similar abnormalities. The processors analyze the signal for matches to library conditions and identify matches that are then used with multiple sensors to localize the source of the matched signal. This process is repeated for each identified signal both by identified matches and for multiple locations. The collection of all the localized and identified data is processed by the image database for presentation. The digitized input signal is also stored in the array data memory for further analysis. Localized features can be further characterized by using beam forming techniques. In the beam forming mode multiple sensors are used to "look" at the same location and the resulting signals analyzed for additional information. Beam forming can be used to shift or scan the region of interest to further characterized and localize the feature.

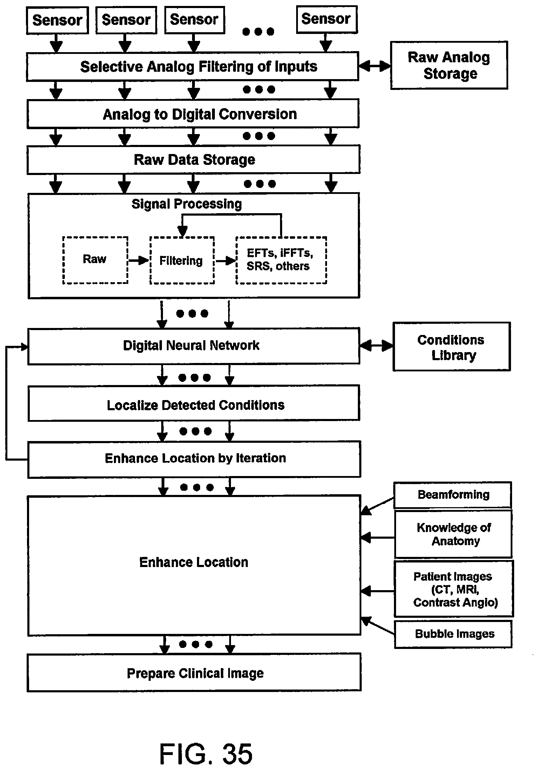

Some of the features of the system as represented in Figure 35 are:
- The use of noise cancellation.
- The elimination of overriding signals.
- The localizers for each signal type.
- The use of the raw data memory.
- The CART data mining software or causal network.
- The patient factors input and how it is used.
- The image database.
- The image.
- The dynamic filtering and its purpose.

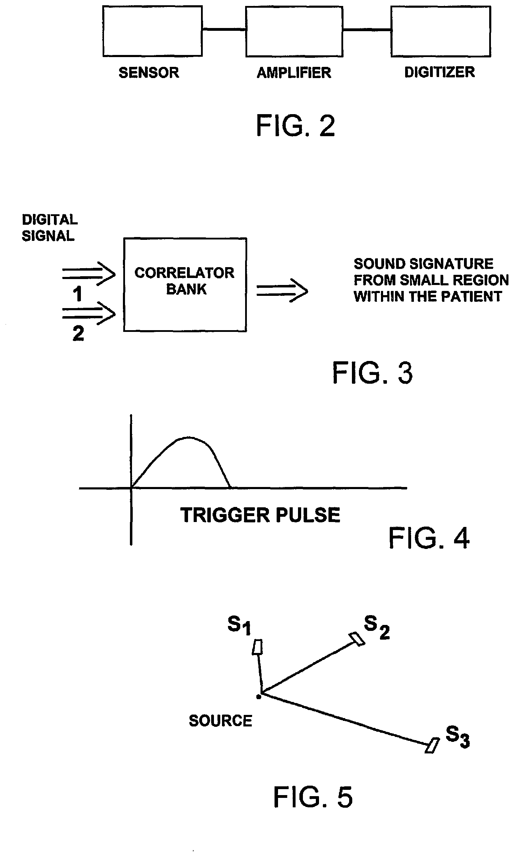

Figure 2 is a more detailed block of the sensor and data acquisition block in Figures 1A and 1B. The sensor signal is amplified and possibly filtered, sometimes dynamically, before being amplified and finally digitized. The signal from each of the sensors in Figure 1A are digitized maintaining the time relationship between each sensor.

Figure 3 shows a block diagram of the correlator bank that localizes the signals. All the sensor signals, with their timing information, are fed into to the correlator bank. The correlator determines, by changing the time relationship between different sensor signals, when two signals are from the same source and record those time relationships.

Multipath rejection via modeling of the signal phase delay of the skull (or body) may be used to improve accuracy of the algorithms.

The relative attenuation of the intervening materials may also be used.

The signal that is common to the multiple sensor signals is recorded as the signal that was created at the location that is determined by the time differences between the arrival of the signal at each sensor, as will be further explained below.

Figure 4 shows a simple trigger pulse test source signal in amplitude versus time.

Figure 5 shows a diagram of the location of a signal source and three sensors, S₁, S₂ and S₃, shown at different distances from the signal source.

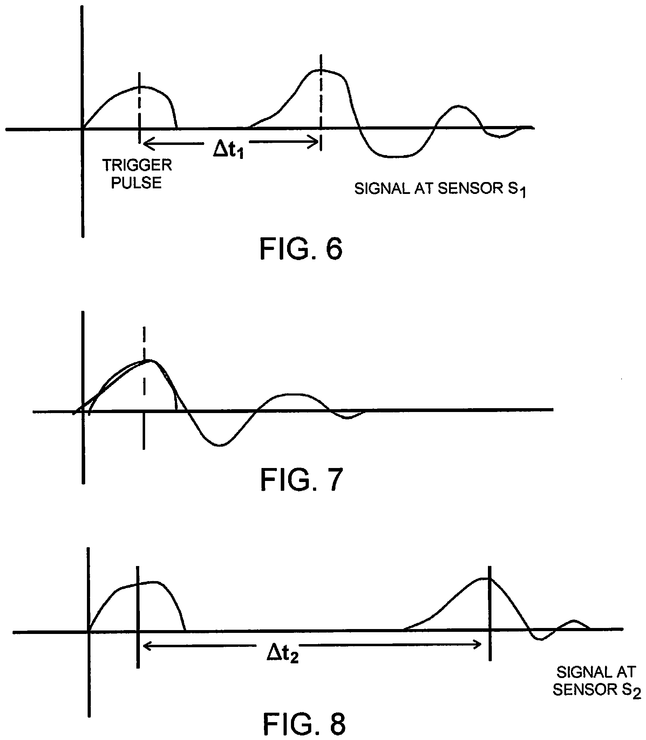

Figure 6 shows a time vs. amplitude waveform of the trigger pulse and the arrival of the signal at sensor S₁. The time that the signal takes to arrive at sensor S₁ is delta t₁.

Figure 7 shows the superposition of the two signals in Figure 6 translated in time by delta t₁.

Figure 8 shows the same data as Figure 6 but for sensor S₂ that is farther from the source than sensor S₁. The time for the signal to arrive from the source at sensor S₂ is delta t₂.

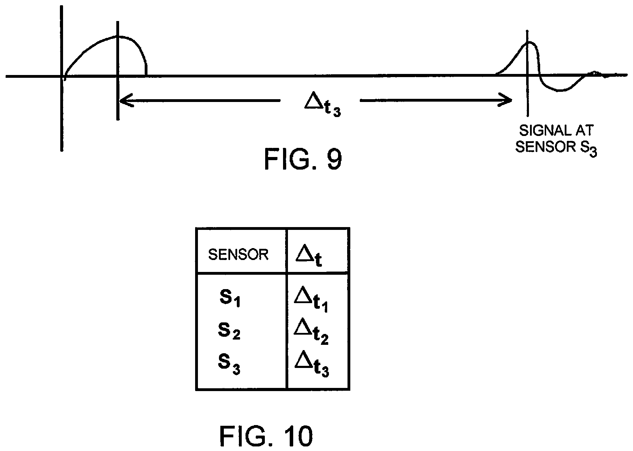

Figure 9 shows that same data as Figures 6 and 8 but for sensor S₃ with a transit time of delta t₃.

Figure 10 is a table with the arrival time for the signal for each of the sensors, S₁, S₂ and S₃.

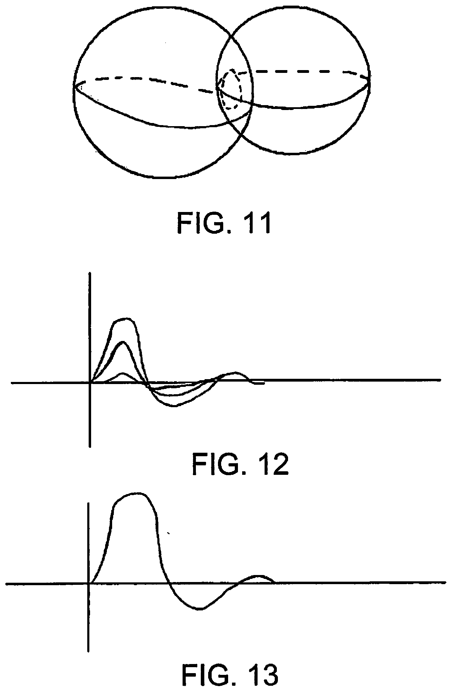

Figure 11 shows two different radius intersecting spheres with the dotted line showing the locus of points where the distance between the centers of sphere 1 and sphere 2 are constant. This locus of points represents the possible position of the source based on only the signal from two sensors. Adding a third sensor, not shown, would restrict the location of the source to a single location, thus fixing its location with respect to the three sensors. Figure 12 shows all the sensor signals from Figures 6, 8 and 9 translated by the times listed in Figure 10.

Figure 13 shows the sum of the three signals shown in Figure 12.

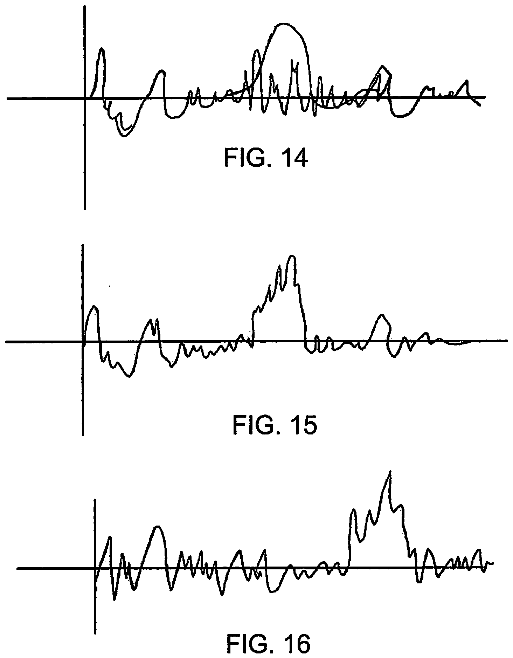

Figure 14 shows the typical signal noise that might be expected to be present on the sensor signal along with the sensor signal as shown in Figure 6.

Figure 15 shows how the sensor signal in Figure 14 would look for a typical signal noise environment.

Figure 16 shows the same signal as Figure 15 but for sensor S₂ rather than sensor S₁.

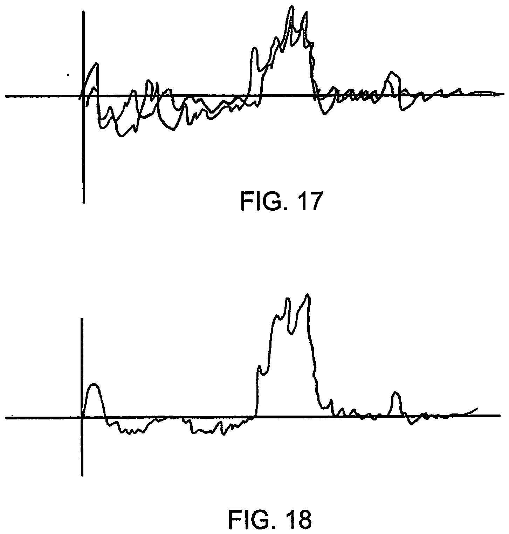

Figure 17 schematically shows superposition of the noisy signals from all the sensors, translated by the times shown in the table in Figure 10.

Figure 18 shows the sum of all the signals in Figure 17 with the noise reduced due to the summing of random noise and the signal increased by the superposition of the common signal.

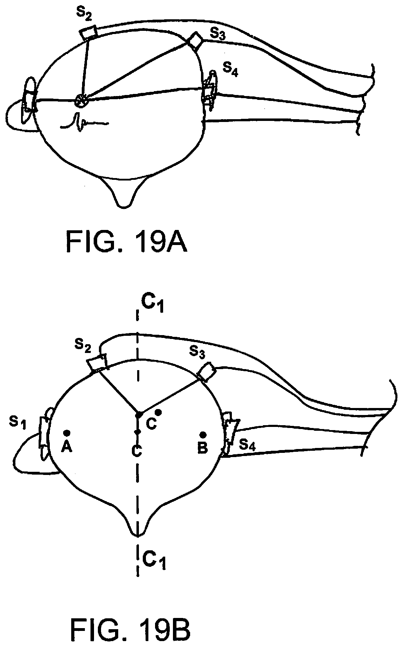

Figure 19A is a pictorial plan view diagram of a patient with sensors S₁ through S₄ attached to the head. A signal source at X is located at distances D₁ through D₄ from the respective sensors. A waveform is shown at X depicting the acoustic signature that is emitted at location X.

Figure 19B is a schematic pictorial diagram of a patient as in Figure 19A. Four locations within the head of the patient are shown; points A, B C and C'. A centerline of symmetry is shown with point C' lying on the centerline and equal distance from sensors S₂ and S₄. Point C is also on the centerline and a short distance from point C'. Small changes in the distances D₂ and D₃ represent the small distance from C' to C. Beamforming techniques depend on changing the phase relationship between data collected by the sensors in a precise and controlled way, such as represented by the example in Figure 19B.

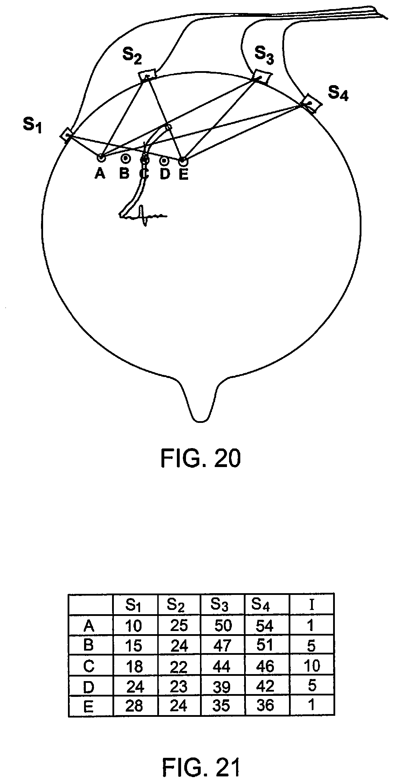

Figure 20 shows a pictorial diagram of a patient with sensors S₁ through S₄ as is shown in Figure 19A with the additional points A-E shown. Points A through E represent small steps across a point of interest, as depicted by a segment of vessel shown across the points A- E. The distances from each point A - E to each sensor S₁ - S₄ are different and are shown in the table in Figure 21.

An example would be when giving tPA to dissolve a clot. The clot region could be monitored and tPA administered while watching the blood flow and perfusion so that the right amount of tPA is administered. This would be possible even if the patient were not able to indicate physical improvements, i.e., asleep or in a natural or induced coma.

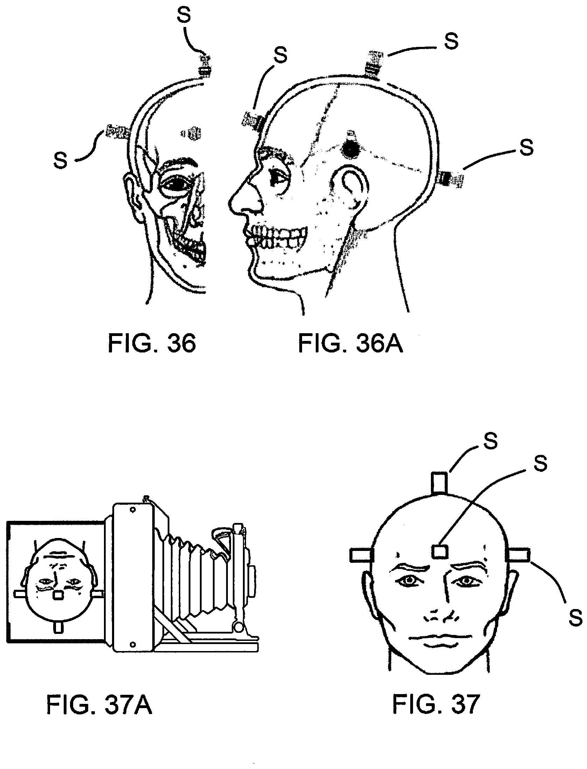

Figures 36 through 37A schematically show methods and devices for establishing the location of the sensors S on the patient's head, to establish these reference points for accurate computation of locations of brain anomalies or conditions. In Figures 36 and 36A the sensors S are referenced to known landmarks of facial features, such as the ears, eyes and nose. The facial landmarks are merged with a CT (or other image) of the patient's head showing the vasculature. Thus, the vessels of the brain are located in relationship to sensors. Figures 37 and 37A indicate the use of a photographic image to accurately locate the reference positions of the sensors. Images can be taken from front, rear, each side and from above the patient, and these images can be merged with known data on the shape of the human head and the cranial vasculature, to arrive at virtual images showing the location of the sensors relative to the cranium and vasculature, as accurate reference points for these pressure pulse receivers. Markers can be placed on the patient's head, to be visible in the photographic images, for the purpose of locating the sensors or accelerometers for reference. Each of the sensors can carry such a visual marker, which can include orthogonal directions.

Taking diagnostic data from the process of the disclosure and merging it with either a reference anatomy or with the patient's own anatomy allows realization of location for detected anomalies. Certain anomalies, ones that are vascular and focal (small extent), can benefit from aligning the anomaly to the vascular structure. For example an aneurysm only exists on the vasculature, primarily on arteries. Further if an aneurysm is detected it will be on a major artery due to detection size limits. Therefore a detected aneurysm will not exist in the space between arteries but on an artery. This fact will allow intelligent placement of the detected anomaly.

One approach to merging the image data with the diagnosis includes using an atlas of the human cranial vasculature and using data from the described localization of a brain anomaly, and placing that anomaly on the atlas image. To further improve the placement of the detected anomaly the atlas can be corrected for the patient's anatomy by imaging the patient with the sensors in place, each sensor having an optical fiducial marker attached. Optically imaging the patient from three axes allows the reference atlas to be expanded or contracted in each axis according to the patient's anatomy.

To further improve the placement of cerebral anomalies an actual CT scan of the patient can be referenced to the patient's skull using facial features on the patient and on the CT scan (Brainlab GmbH, Munich, Germany). Once that alignment has been completed the sensors can be referenced to the CT scan allowing placement of the detected anomalies on the actual anatomy of the patient.

The apparatus of the invention also enable a detection of concussion. This does not involve any identifiable anomaly in the blood vessels or in tissue between a blood vessel and a sensor of the invention. It does, however, involve a signature, or a family of signatures, to which pressure pulse signals emanating from the vessels can be matched, or substantially or approximately matched to an approximate degree of certainty.

The condition of concussion, caused by a blow to the head of a patient, is not completely understood. There are different degrees of concussion, the milder of which usually require rest for recuperation, and the more severe of which can lead to serious brain injuries and which might require treatment such as relief of pressure on the brain.

According to the invention, when a patient has suffered a blow to the head that may have caused concussion, at least one of the sensors of the equipment described above (and preferably more, or all) are secured on the head. Pressure pulse readings are taken from all sensors, which are accelerometers. If concussion is present (at least concussion above a very mild level), readings on at least some of the sensors, and sometimes all sensors, will indicate concussion when compared with a library of concussion signatures (with an approximately known degree of certainty). Such a library has been developed by the inventors herein, based on testing of numerous subjects known to have concussion in ranges from mild to moderate, and on testing of non-concussion subjects for control. Also notable is that the system of the invention can detect a patient's level of concussion in most instances, from mild to severe and determine if the patient has recovered from the concussion. According to the invention, the data are analyzed in the frequency domain.

Cerebral vasospasm is a dangerous condition that generally occurs due to a ruptured aneurysm. A typically strong candidate for vasospasm is a patient who has suffered subarachnoid stroke, usually from aneurysm. The abnormal presence of a substantial amount of blood on the outer or subarachnoid surface of a blood vessel can cause a reaction in the vessel by which the vessel constricts and over-contracts, and can sometimes completely shut down the flow of blood through the vessel. Traumatic brain injury can also produce blood in the subarachnoid space and trigger vasospasm.

Following a subarachnoid stroke, a patient is often required to spend days or weeks in hospital critical care waiting for the occurrence of a cerebral vasospasm, so that the condition can be tracked by physicians.

The invention facilitates detection of vasospasm immediately as it occurs. The patient's "BrainPulse" signature, as obtained by exemplary configurations, will detect vasospasm so that therapeutic measures can be taken such as angioplasty, placement of stents or use of vasospasm suppressors. The BrainPulse signature for vasospasm is distinctly different from other signatures of anomalies as discussed above. The term "BrainPulse" as used herein refers to a unique signature of the brain of each patient, which will differ for the patient under different conditions and anomalies of the brain, as detected by the system and method of the invention using accelerometers and signal analysis. The BrainPulse is an average of skull movements gated by the heart rate; it changes shape with respiration so averaging over several respiration cycles produces a smooth average. Typically 45 heartbeats comprise an appropriate average.

For vasospasm the acceleration data are analyzed essentially in a way to create a ratio of diastolic pulsing strength to systolic pulsing strength in the brain tissue, a DS ratio. A low DS ratio tends to indicate vasospasm. This can be done by taking and processing acceleration signal data from sensors at the front of the head and/or the back of the head. Data from side and coronal sensors do not correlate well with vasospasm indication.

It is believed that total acceleration due to blood flow during diastole will be reduced in comparison to systole, in a patient exhibiting vasospasm, as compared to a normal patient. Basically the DS ratio is determined as a ratio between total skull acceleration during diastole and total acceleration during systole.

In one algorithm for this purpose the wave form data over a series of pulses, in the time domain, are "rubberbanded" (see below) to correlate data in phase. The wave form is then rectified, to look at absolute value of amplitude at each point in the wave form. In one test the wave form was 4096 channels, over a selected time period represented by those channels. After normalizing the wave form data by subtracting from each wave form point the average value of all points, area under the wave form curve (or a summation of the value of all points) for a diastolic portion was compared to area under the curve (or summation) for a systolic portion. These area values are assumed to indicate relative strength of pulsing in diastole and systole. For purposes of this example the one-third channel point was assumed to be the division between systole and diastole, i.e channel 1365 in this case. This is somewhat arbitrary and will vary for different patients and is dependent on other factors including heart rate, but even in this simplified procedure a strong correlation to vasospasm condition can be found.

The results were that a strong correlation was noted between the DS ratio value and the patient's vasospastic/non-vasospastic condition. For example, a normal patient had a ratio of 4.26, vs. a severely vasospastic patient with a ratio of 1.27. Other arithmetic procedures/algorithms could be used to create a DS ratio reflecting diastolic pulsing strength vs. systolic pulsing strength.

The presently disclosed configuration involves averaging of series of wave forms, such as a series of data taken over 10 or 30 or 50 heartbeat pulses, for example. Since heart rate can vary considerably within such a series, the wave forms are of differing time durations and must be correlated in a way that averages the corresponding features of each wave form, rather than simply generating noise by adding together a number of phase-shifted non-time-correlated wave forms. Pursuant to the exemplary configuration the wave forms are objected to a "rubberbanding" technique. A plurality of repetitive similar wave forms are obtained in the time domain, and these can be accelerometer signal data over a series of heartbeats. The wave form data is overlaid and averaged, but to effect this, similar segments of different wave forms are matched, and the averaging is optimized by shifting a plurality of the wave forms in phase, including stretching or contracting the time duration of a wave form or wave form segment. Phase shifting of the wave forms is continued in small increments to correlate the wave forms, until the Fourier transform of the resulting average spectrum results in a maximum signal to noise ratio. Thus, repeated averaging-in of wave forms of a series of repetitive similar wave forms will cause noise to be substantially eliminated from the resulting spectrum. Although these wave forms will typically be from a series of consecutive wave forms, they can also be drawn from a non-consecutive plurality of wave forms.

The preferred embodiments described above are intended to illustrate the principles of the invention, but not to limit its scope. Other embodiments and variations to these preferred embodiments will be apparent to those skilled in the art and may be made without departing from the scope of the disclosure. The scope of protection is solely defined by the appended claims.

## Claims

1. A system for noninvasively detecting heartbeat-induced repeated cranial accelerations for detecting concussion of the brain of a patient, comprising:
at least one accelerometer adapted for positioning against the head of a patient at a selected location so as to be directed toward the brain, and adapted for sensing and measuring acceleration of the skull occurring from heartbeat-induced repeated pulsing of blood flow into the vasculature of the brain and resultant expansion and contraction of the blood vessels which move the brain tissue due to such pulsing,
a digitizer connected to the accelerometer and adapted to receive accelerometer signals and convert the signals to digitized data, by subjecting the accelerometer signals to a Fourier transform to obtain a frequency domain of the accelerometer signals, and
a computer with a comparator, the comparator being connected to the digitizer and adapted for recognizing in said digital data any of a plurality of particular data signatures representing brain conditions as read by the accelerometer, wherein said digital data is compared with a library of characteristics of signals acquired from patients known to have concussion and matches of said digital data to said signals are found.

2. The system of claim 1, wherein associating said signal data is limited to signal data at a frequency of 500 Hz or lower.

3. The system of claim 1, wherein associating said signal data is limited to signal data at a frequency of 100 Hz or lower.

4. The system of claim 1, wherein the step of associating said signal data is limited to signal data at a frequency of 30 Hz or lower.

5. The system of claim 1, wherein the computer is adapted to derive features from the accelerations, indicative of a brain condition, to detect frequency intensity at a range of harmonics of the heart rate below about 100 Hz to use these harmonics and ratios, sums, differences and products of these harmonics singly or in combination to determine brain anomalies.

6. The system of claim 6, wherein deriving features comprises detecting frequency intensity at a range of harmonics of the heart rate below about 30 Hz and using sums and ratios of these harmonics to determine concussion.

## Patentansprüche

1. System zum nichtinvasiven Erkennen von herzschlaginduzierten wiederholten kranialen Beschleunigungen zum Erkennen von Erschütterungen des Gehirns eines Patienten, umfassend:
wenigstens einen Beschleunigungsmesser, der für die Positionierung gegen den Kopf eines Patienten an einer ausgewählten Stelle angepasst ist, um in Richtung des Gehirns gerichtet zu sein, und zum Erfassen und Messen der Beschleunigung des Schädels angepasst ist, die durch das herzinduzierte wiederholte Pulsieren des Blutflusses in die Gefäße des Gehirns und der daraus resultierenden Ausdehnung und Kontraktion der Blutgefäße auftritt, die das Hirngewebe aufgrund dieses Pulsierens bewegen,
einen Digitalisierer, der mit dem Beschleunigungsmesser verbunden und angepasst ist, um Beschleunigungsmessersignale zu empfangen und die Signale in digitalisierte Daten umzuwandeln, indem die Beschleunigungsmessersignale einer Fourier-Transformation unterzogen werden, um einen Frequenzbereich der Beschleunigungsmessersignale zu erhalten, und
einen Computer mit einem Komparator, wobei der Komparator mit dem Digitalisierer verbunden ist und zum Erkennen in den digitalen Daten einer beliebigen aus einer Mehrzahl von bestimmten Datensignaturen angepasst ist, die Gehirnzustände darstellen, wie sie von dem Beschleunigungsmesser gelesen werden, wobei die digitalen Daten mit einer Bibliothek von Merkmalen von Signalen verglichen werden, die von Patienten erhalten wurden, von denen bekannt ist, dass sie eine Gehirnerschütterung haben, und Übereinstimmungen der digitalen Daten mit diesen Signalen gefunden werden.

2. System nach Anspruch 1, wobei das Zuordnen der Signaldaten auf Signaldaten mit einer Frequenz von 500 Hz oder weniger beschränkt ist.

3. System nach Anspruch 1, wobei das Zuordnen der Signaldaten auf Signaldaten mit einer Frequenz von 100 Hz oder weniger beschränkt ist.

4. System nach Anspruch 1, wobei der Schritt des Zuordnens der Signaldaten auf Signaldaten mit einer Frequenz von 30 Hz oder weniger beschränkt ist.

5. System nach Anspruch 1, wobei der Computer angepasst ist, um Merkmale aus den Beschleunigungen abzuleiten, die auf einen Gehirnzustand hinweisen, um die Frequenzintensität in einem Bereich von Oberschwingungen der Herzfrequenz unter etwa 100 Hz zu erfassen, um diese Oberschwingungen und Verhältnisse, Summen, Differenzen und Produkte dieser Oberschwingungen einzeln oder in Kombination zu verwenden, um Gehirnanomalien zu bestimmen.

6. System nach Anspruch 6, wobei das Ableiten von Merkmalen das Erkennen einer Frequenzintensität in einem Bereich von Oberschwingungen der Herzfrequenz unter etwa 30 Hz und das Verwenden von Summen und Verhältnissen dieser Oberschwingungen umfasst, um eine Gehirnerschütterung zu bestimmen.

## Revendications

1. Système de détection non effractive d'accélérations crâniennes répétées induites par battement de cœur, pour détecter une commotion cérébrale d'un patient, comprenant :
au moins un accéléromètre adapté pour le positionnement contre la tête d'un patient à un emplacement sélectionné afin d'être dirigé vers le cerveau, et adapté pour détecter et mesurer une accélération crânienne due à des impulsions répétées induites par battement de cœur du débit sanguin dans la vascularisation cérébrale et une expansion et une contraction résultantes des vaisseaux sanguins qui déplacent le tissu cérébral en raison de telles impulsions,
un numériseur connecté à l'accéléromètre et adapté pour recevoir des signaux d'accéléromètre et convertir les signaux en données numérisées, en soumettant les signaux d'accéléromètre à une transformée de Fourier pour obtenir un domaine fréquentiel des signaux d'accéléromètre, et
un ordinateur avec un comparateur, le comparateur étant connecté au numériseur et adapté pour reconnaître, dans lesdites données numériques, de quelconques d'une pluralité de signatures de données particulières représentant des conditions cérébrales telles que lues par l'accéléromètre, dans lequel lesdites données numériques sont comparées à une bibliothèque de caractéristiques de signaux acquis de patients dont l'état commotionnel est connu et des correspondances desdites données numériques auxdits signaux sont trouvées.

2. Système de la revendication 1, dans lequel l'association desdites données de signal est limitée à des données de signal à une fréquence de 500 Hz ou moins.

3. Système de la revendication 1, dans lequel l'association desdites données de signal est limitée à des données de signal à une fréquence de 100 Hz ou moins.

4. Système de la revendication 1, dans lequel l'étape de l'association desdites données de signal est limitée à des données de signal à une fréquence de 30 Hz ou moins.

5. Système de la revendication 1, dans lequel l'ordinateur est adapté pour déduire des particularités des accélérations, indicatives d'une condition cérébrale, pour détecter une intensité fréquentielle dans une gamme d'harmoniques de la fréquence cardiaque inférieures à 100 Hz pour utiliser ces harmoniques et des rapports, des sommes, des différences, et des produits de ces harmoniques individuellement ou en association pour déterminer des anomalies cérébrales.

6. Système de la revendication 6, dans lequel la déduction de particularités comprend la détection d'une intensité fréquentielle dans une gamme d'harmoniques de la fréquence cardiaque inférieures à 30 Hz et l'utilisation de sommes et de rapports de ces harmoniques pour déterminer une commotion.
